# FASCICULE DE BREVET EUROPEEN

(11) **EP 0 719 764 B1**
(45) Date de publication et mention de la délivrance du brevet: **04.11.1998**
(21) Numéro de dépôt: 95402842.9
(22) Date de dépôt: 18.12.1995
(51) Int. Cl.: C07D 217/24, A61K 31/47

(54) **Dérivés de 3-phénylisoquinoléin-1(2H)-one comme ligands des récepteurs benzodiazépiniques associés aux récepteurs GABAA**
3-Phenylisochinolin-1(2H)-one-Derivate als Liganden der mit GABAA Rezeptoren verbundenen Benzodiazepine Rezeptoren
3-Phenylisoquinolin-1(2H)-one derivatives as ligands of benzodiazepine receptors associated with GABAA receptors

(30) Priorité: 29.12.1994 FR 9415837
(43) Date de publication de la demande: 03.07.1996
(73) Titulaire: SYNTHELABO, 92350 Le Plessis Robinson (FR)
(72) Inventeur: Sevrin, Mireille, F-75014 Paris (FR); Marabout, Benoit, F-91380 Chilly Mazarin (FR); Froissant, Jacques, F-41160 Moree (FR); Guinot, Catherine, F-75019 Paris (FR)
(74) Mandataire: Ludwig, Jacques

## Description

La présente invention a pour objet des dérivés de 3-phénylisoquinoléin-1(2*H*)-one, leur préparation et leur application en thérapeutique.

Les composés de l'invention répondent à la formule générale (I) 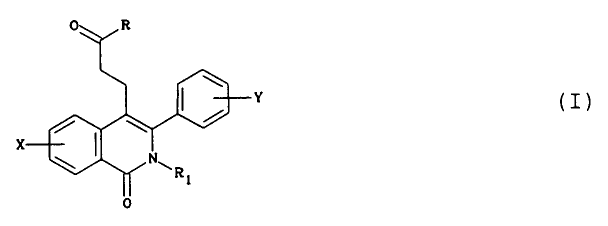 dans laquelle
X représente un atome d'hydrogène, un atome d'halogène, un groupe trifluorométhyle, un groupe alkyle en C₁-C₃, ou un ou deux groupes alcoxy en C₁-C₃,
Y représente un atome d'hydrogène, un atome d'halogène, un groupe alkyle en C₁-C₃ ou un groupe alcoxy en C₁-C_{3,}
R₁ représente un groupe alkyle en C₁-C₄, et
R représente un groupe hydroxy, un groupe méthoxy, un groupe éthoxy, ou un groupe de formule générale NR₂R₃ dans laquelle
R₂ et R₃ représentent chacun un atome d'hydrogène ou un groupe alkyle en C₁-C₄.

Les composés préférés de l'invention sont ceux dans la formule desquels R₁ représente un groupe méthyle et R représente un groupe méthylamino.

Conformément à l'invention, on peut préparer les composés de formule générale (I) par un procédé illustré par le schéma qui suit.

On soumet une 3-phénylisoquinoléin-1(2*H*)-one de formule générale (II), dans laquelle X, Y et R₁ sont tels que définis ci-dessus, à un traitement par l'oxychlorure de phosphore dans un solvant tel que le *N*,*N*-diméthylformamide, à une température de 80 à 120°C, pour obtenir, après hydrolyse, l' aldéhyde de formule générale (III), puis on traite ce dernier avec le (diméthoxyphosphinyl)acétate de méthyle, dans un solvant tel que le tétrahydrofurane, à une température de

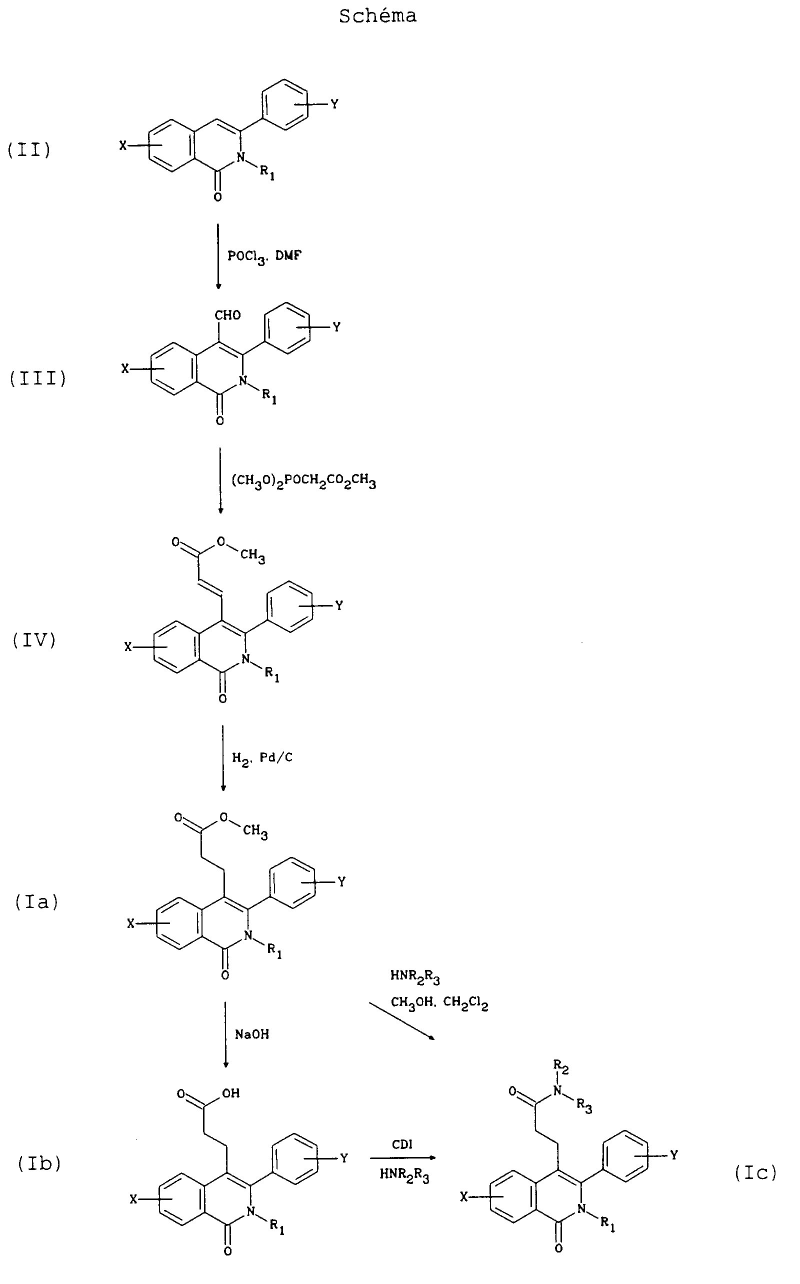

20 à 67°C, pour obtenir l'ester méthylique de formule générale (IV), puis on soumet ce dernier à une hydrogénation, par exemple en présence de charbon palladié, pour aboutir à l'ester de formule générale (Ia), laquelle correspond à la formule générale (I) lorsque R représente un groupe méthoxy.

Si l'on désire un composé de formule générale (I) où R représente un groupe hydroxy, on soumet l'ester de formule générale (Ia) à une hydrolyse en milieu basique, dans un solvant tel que l'éthanol ou le méthanol, à une température de 60 à 80°C, pour obtenir un composé de formule générale (Ib) et, lorsque on désire un composé de formule générale (I) où R représente un groupe de formule NR₂R₃ on peut soit traiter l'ester de formule générale (Ia) avec une amine de formule générale HNR₂R₃, à température ambiante et dans un solvant tel qu'un mélange de méthanol et de dichlorométhane, soit traiter l'acide de formule générale (Ib) avec cette amine en passant par l'imidazolide intermédiaire préparé *in situ* au moyen de *N,N*'-carbonyldiimidazole.

Pour préparer les composés de formule générale (I) où R représente un groupe éthoxy, on soumet l'acide de formule générale (Ib) à l'action du chlorure de thionyle dans l'éthanol, à une température de 20 à 65°C.

Les composés de départ, de formule générale (II), sont connus et peuvent être préparés par des méthodes analogues à celles décrites dans *Synthesis* (1982) 329-330, *Synthesis* (1980) **10** 845-847, *CA* **79**(15) 91351k et *CA* **76**(1) 3666b. Les intermédiaires de formules générales (III) et (IV) sont connus et décrits dans la demande de brevet EP-0 424 929.

Les exemples qui vont suivre illustrent la préparation de quelques composés de l'invention. Les microanalyses élémentaires, et les spectres I.R. et R.M.N. confirment les structures des composés obtenus. Les numéros indiqués entre parenthèses dans les titres des exemples correspondent à ceux de la 1ère colonne du tableau 1 donné plus loin. Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°2)

### 2-Méthyl-1-oxo-3-phényl-1,2-dihydroisoquinoléine-4-propan₋oate de méthyle.

### 1.1. 2-Méthyl-1-oxo-3-phényl-1,2-dihydroisoquinoléine-4-carboxaldéhyde.

On refroidit à 0°C, sous atmosphère d'argon, 80 ml de *N,N*-diméthylformamide sec, puis on ajoute, goutte à goutte, 6,8 ml (71,7 mmoles) d'oxychlorure de phosphore et on agite le mélange à température ambiante pendant 1h.
On ajoute 5 g (21,25 mmoles) de 2-méthyl-3-phénylisoquino₋léin-1(2H)-one en solution du dichloro-1,2-éthane, on chauffe progressivement le mélange à 120°C et on le maintient à cette température pendant 4h.
On le refroidit à température ambiante, on évapore le solvant sous pression réduite, on ajoute 200 ml d'éther diét₋hylique et de la glace au résidu, on ajoute de la soude 1N, on sépare la phase organique et on extrait la phase aqueuse avec 200 ml de dichlorométhane.
On réunit les deux phases organiques, on les sèche sur sul₋fate de sodium, on les filtre et on évapore les solvants sous pression réduite.
On purifie le produit obtenu par chromatographie sur colonne de gel de silice en éluant avec un mélange de cyclohexane et de dichlorométhane allant de 80/20 à 50/50, puis avec un mélange de dichlorométhane et d'acétate d'éthyle allant de 100/0 à 70/30.
Après recristallisation dans le cyclohexane on obtient 3,93 g (14,93 mmoles) de solide blanc.
Point de fusion : 138-141°C.

### 1.2. (E)-3-(2-Méthyl-1-oxo-3-phényl-1,2-dihydroisoquino₋léin-4-yl)prop-2-énoate de méthyle.

Dans un ballon de 500 ml placé sous atmosphère d'argon on introduit 0,9 g d'hydrure de sodium en suspension à 60% dans l'huile (22,5 mmoles), on le lave au pentane et on le met en suspension dans 150 ml de tétrahydrofurane sec.
On refroidit le mélange à 0°C avec un bain de glace, on ajoute, goutte à goutte, 3,4 g (18,7 mmoles) de (diméthoxy₋phosphinyl)acétate de méthyle en solution dans 10 ml de tétrahydrofurane et on agite le mélange pendant 30 min à température ambiante.
On ajoute ensuite 4 g (15,2 mmoles) de 2-méthyl-1-oxo-3-phé₋nyl-1,2-dihydroisoquinoléine-4-carboxaldéhyde, on chauffe progressivement jusqu'au reflux du tétrahydrofurane et on maintient le mélange à cette température pendant 6h.
On le refroidit à 0°C avec un bain de glace, on ajoute quelques gouttes de méthanol pour neutraliser l'excès d'hydrure, et on évapore les solvants sous pression réduite. On ajoute au résidu 250 ml de dichlorométhane et de l'eau glacée, on sépare la phase organique, on la lave à l'eau, on la sèche sur sulfate de sodium, on la filtre, on évapore le solvant sous pression réduite et on purifie le résidu par chromatographie sur colonne de gel de silice en éluant avec un mélange de dichlorométhane et d'acétate d'éthyle allant de 100/0 à 70/30. On recristallise le produit dans un mélange de cyclohexane et de dichlorométhane pour obtenir 3,84 g (12,02 mmoles) de solide blanc.
Point de fusion : 184-185°C.

### 1.3. 2-Méthyl-1-oxo-3-phényl-1,2-dihydroisoquinoléine-4-propanoate de méthyle.

A une solution de 3,8 g (11,9 mmoles) de (E)-3-(2-méthyl-1-oxo-3-phényl-1,2-dihydroisoquinoléin-4-yl)prop-2-énoate de méthyle dans 100 ml d'acide acétique on ajoute 0,25 g de charbon palladié à 5% et on soumet la suspension à une hydrogénation dans un appareil de Parr sous une pression d'environ 0,3 MPa pendant 1h à température ambiante puis pendant 3h à environ 50°C.
On élimine le catalyseur par filtration, on concentre le filtrat sous pression réduite, on ajoute au résidu de l'eau glacée, 200 ml de dichlorométhane et 100 ml de soude 1N, on sépare la phase organique, on la lave à l'eau, on la sèche sur sulfate de sodium, et on évapore le solvant sous pression réduite.
On purifie le produit brut par chromatographie sur colonne de gel de silice en éluant avec un mélange de dichloromé₋thane et d'acétate d'éthyle allant de 100/0 à 70/30. Après recristallisation dans un mélange de cyclohexane et de dichlorométhane on obtient 2,69 g (8,37 mmoles) de solide blanc.
Point de fusion : 134-135°C.

### Exemple 2 (Composé N°1)

### Acide 2-méthyl-1-oxo-3-phényl-1,2-dihydroisoquinoléine-4-propanoïque.

Dans un ballon de 250 ml on dissout 1,2 g (3,7 mmoles) de 2-méthyl-1-oxo-3-phényl-1,2-dihydroisoquinoléine-4-propa₋noate de méthyle dans 80 ml de méthanol, on ajoute 0,25 g (6,25 mmoles) de soude en pastilles et 1 ml d'eau, puis on agite le mélange à température ambiante pendant 1h et au reflux pendant 2h.
On refroidit le mélange, on évapore le solvant sous pression réduite, on reprend le résidu avec 50 ml d'eau et 50 ml d'éther diéthylique et on ajoute, goutte à goutte, de l'acide chlorhydrique à 36%. On collecte l'insoluble par filtration, on le lave à l'eau et on le sèche.
On obtient 0,91 g (2,96 mmoles) de solide blanc.
Point de fusion : 138-140°C.

### Exemple 3 (Composé N°4)

### N,2-Diméthyl-1-oxo-3-phényl-1,2-dihydroisoquinoléine-4-pro₋panamide.

Dans un ballon de 250 ml contenant une solution de 1,25 g (3,85 mmoles) de 2-méthyl-1-oxo-3-phényl-1,2-dihydroisoqui₋noléine-4-propanoate de méthyle dans 20 ml de dichloro₋méthane et 80 ml de méthanol, on fait passer un courant de méthylamine gazeuse jusqu'à saturation, et on laisse le mélange sous agitation à température ambiante pendant 3 jours.
On évapore les solvants sous pression réduite et on purifie le résidu par chromatographie sur colonne de gel de silice en éluant avec un mélange de dichlorométhane et d'acétate d'éthyle allant de 100/0 à 80/20, puis avec un mélange de dichlorométhane et de méthanol 95/5. Après recristallisation dans l'acétonitrile on obtient 0,85 g (2,65 mmoles) de solide cristallin blanc.
Point de fusion : 185-186°C.

### Exemple 4 (Composé N°23)

### N,N,2-Triméthyl-1-oxo-3-phényl-1,2-dihydroisoquinoléine-4-propanamide.

Dans un ballon de 500 ml, placé sous atmosphère d'argon, on prépare une suspension de 0,9 g (2,93 mmoles) d'acide 2-méthyl-1-oxo-3-phényl-1,2-dihydroisoquinoléine-4-propanoïque dans 150 ml de dichlorométhane, on ajoute 0,7 g (4,3 mmoles) de *N,N'*-carbonyldiimidazole, on agite le mélange à température ambiante pendant 2h, on le sature pendant 1 min avec de la diméthylamine gazeuse et on poursuit l'agitation pendant 12h.
On évapore le solvant sous pression réduite, on reprend le résidu avec 200 ml de dichlorométhane, 100 ml d'eau et de l'acide chlorhydrique 1M, on sépare la phase organique, on la lave à l'eau, on la sèche sur sulfate de sodium, on la filtre et on évapore le solvant sous pression réduite. On purifie le résidu par chromatographie sur colonne de gel de silice en éluant avec un mélange de dichlorométhane et d'acétate d'éthyle allant de 100/0 à 70/30, puis avec un mélange de dichlorométhane et de méthanol 95/5. Après recristallisation dans un mélange de dichlorométhane, méthanol et acétonitrile on obtient 0,72 g (2,15 mmoles) de solide blanc.
Point de fusion : 165,5-166°C.

### Exemple 5 (Composé N°10)

### N,2-Diméthyl-7-chloro-1-oxo-3-phényl-1,2-dihydroisoquino₋léine-4-propanamide.

### 5.1. 7-Chloro-2-méthyl-1-oxo-3-phényl-1,2-dihydroisoqui₋noléine-4-carboxaldéhyde.

On refroidit à 0°C, sous atmosphère d'argon, 100 ml de *N,N*-diméthylformamide sec, on ajoute, goutte à goutte, 12 ml (128 mmoles) d'oxychlorure de phosphore et on agite le mélange à température ambiante pendant 1h.
On ajoute 13,7 g (51 mmoles) de 7-chloro-2-méthyl-3-phénylisoquinoléin-1(2*H*)-one, on chauffe progressivement le mélange à 110°C et on le maintient à cette température pendant 4h.
On le refroidit à température ambiante, on évapore le solvant sous pression réduite, on ajoute 200 ml d'éther diéthylique et de la glace au résidu, on ajoute de la soude 1N, on sépare la phase organique et on extrait la phase aqueuse avec 200 ml de dichlorométhane.
On réunit les deux phases organiques, on les sèche sur sulfate de sodium, on les filtre et on évapore les solvants sous pression réduite.
On purifie le résidu par chromatographie sur colonne de gel de silice en éluant avec un mélange de cyclohexane et de dichlorométhane allant de 80/20 à 50/50, puis avec un mélange de dichlorométhane et d'acétate d'éthyle allant de 100/0 à 70/30.
Après recristallisation dans le cyclohexane on obtient 7,68 g (26 mmoles) de solide blanc.
Point de fusion : 172-173°C.

### 5.2. (E)-3-(7-Chloro-2-méthyl-1-oxo-3-phényl-1,2-dihydro₋isoquinoléin-4-yl)prop-2-énoate de méthyle.

Dans un ballon de 500 ml placé sous atmosphère d'argon on introduit 1,3 g d'hydrure de sodium en suspension à 60% dans de l'huile (32,5 mmoles), on le lave au pentane et on le met en suspension dans 200 ml de tétrahydrofurane sec.
On refroidit le mélange à 0°C avec un bain de glace, on ajoute, goutte à goutte, 4,6 ml (28 mmoles) de (diméthoxyphosphinyl)acétate de méthyle en solution dans 10 ml de tétrahydrofurane et on agite le mélange pendant 30 min à température ambiante.
On ajoute ensuite 7,68 g (26 mmoles) 7-chloro-2-méthyl-1-oxo-3-phényl-1,2-dihydroisoquinoléine-4-carboxaldéhyde, on chauffe progressivement jusqu'au reflux du tétrahydrofurane et on maintient le mélange à cette température pendant 3h.
On le refroidit à 0°C avec un bain de glace, on ajoute quelques gouttes de méthanol pour neutraliser l'excès d'hydrure, et on évapore les solvants sous pression réduite.
On ajoute au résidu 250 ml de dichlorométhane et de l'eau glacée, on sépare la phase organique, on la lave à l'eau, on la sèche sur sulfate de sodium, on la filtre, on évapore le solvant sous pression réduite et on purifie le résidu par chromatographie sur colonne de gel de silice en éluant avec un mélange de dichlorométhane et d'acétate d'éthyle allant de 100/0 à 70/30. On recristallise le produit dans un mélange de cyclohexane et de dichlorométhane pour obtenir 8,35 g (23,6 mmoles) de solide blanc.
Point de fusion : 185-188°C.

### 5.3. 7-Chloro-2-méthyl-1-oxo-3-phényl-1,2-dihydro₋isoquinoléine-4-propanoate de méthyle.

A une solution de 8,35 g (23,6 mmoles) de (*E*)-3-(7-chloro-2-méthyl-1-oxo-3-phényl-1,2-dihydroisoquinoléin-4-yl)prop-2-énoate de méthyle dans 150 ml d'acétate d'éthyle on ajoute 0,8 g d'oxyde de platine et on soumet la suspension à une hydrogénation dans un appareil de Parr sous une pression d'environ 0,3 Mpa pendant 5h à température ambiante.
On élimine le catalyseur par filtration et on concentre le filtrat sous pression réduite.
On purifie le produit brut par chromatographie sur colonne de gel de silice en éluant avec un mélange de cyclohexane et de dichlorométhane allant de 50/50 à 0/100, puis avec un mélange de dichlorométhane et d'acétate d'éthyle allant de 100/0 à 90/10. Après évaporation des solvants sous pression réduite on reprend le résidu dans du cyclohexane pour obtenir 4,73 g (13,3 mmoles) de solide blanc.
Point de fusion : 143-144°C.

### 5.4. N,2-Diméthyl-7-chloro-1-oxo-3-phényl-1,2-dihydroisoquinoléine-4-propanamide.

Dans un ballon de 250 ml contenant une solution de 1,7 g (4,78 mmoles) de 7-chloro-2-méthyl-1-oxo-3-phényl-1,2-dihydroisoquinoléine-4-propanoate de méthyle dans 20 ml de dichlorométhane et 80 ml de méthanol on fait passer un courant de méthylamine gazeuse jusqu'à saturation, et on laisse le mélange sous agitation à température ambiante pendant 4 jours.
On évapore les solvants sous pression réduite et on purifie le résidu par chromatographie sur colonne de gel de silice en éluant avec un mélange de dichlorométhane et d'acétate d'éthyle allant de 100/0 à 80/20, puis avec un mélange de dichlorométhane et de méthanol 95/5.
Après recristallisation dans l'acétate d'éthyle on obtient 1,36 g (3,83 mmoles) de solide blanc.
Point de fusion : 161-162°C.

Le tableau qui suit illustre les structures chimiques et les propriétés physiques de quelques composés de formule générale (I).

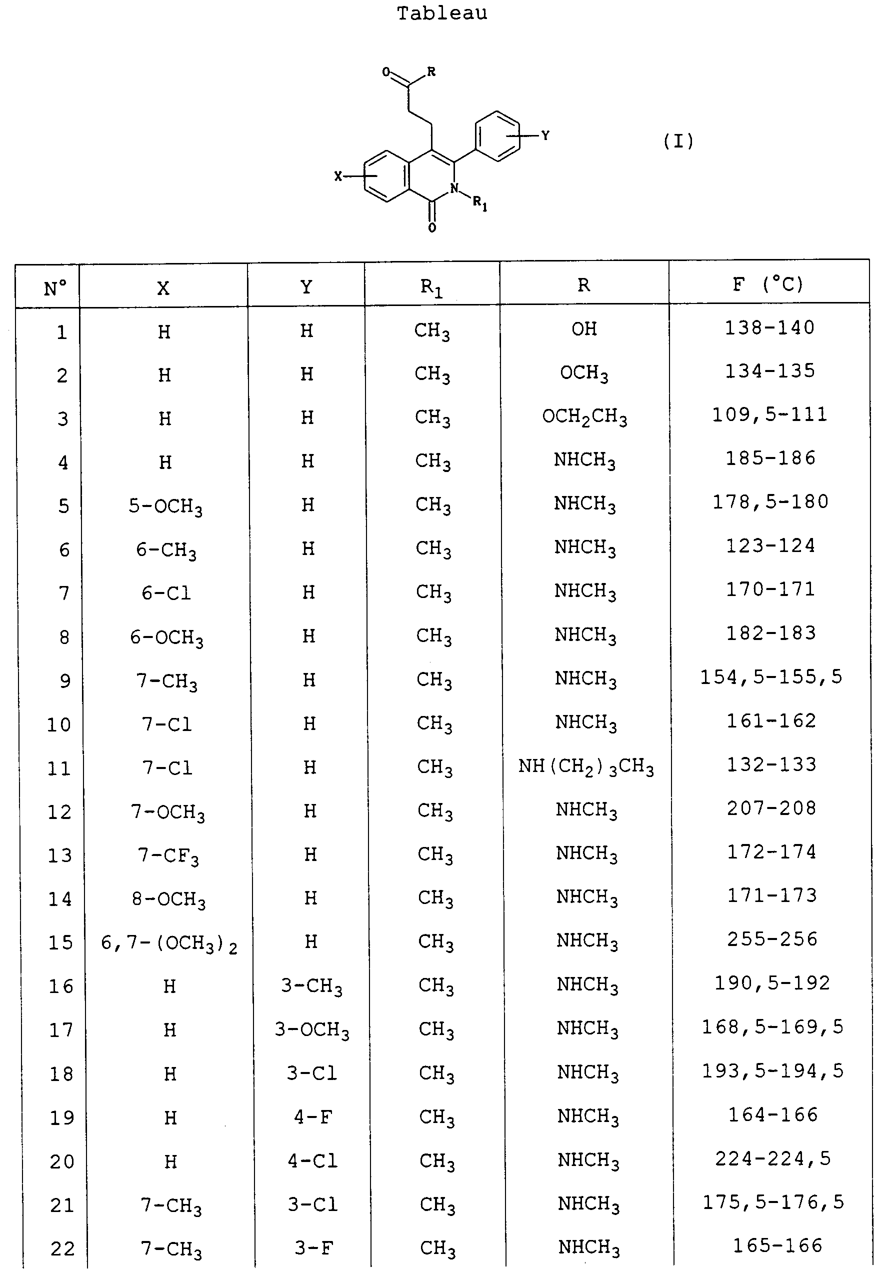

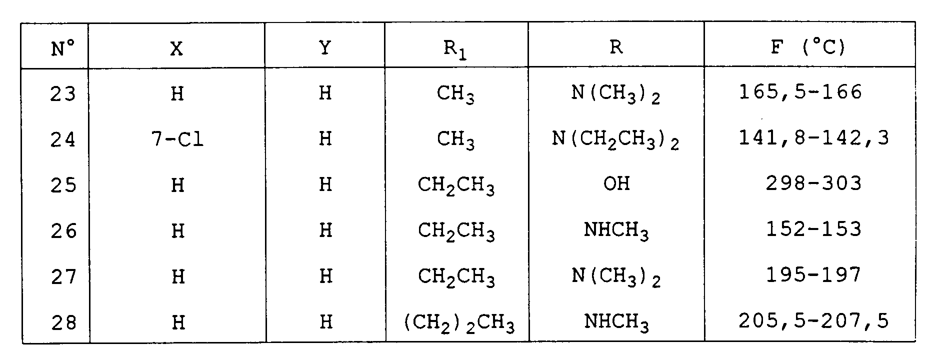

Les composés de l'invention ont été soumis à des essais pharmacologiques qui ont mis en évidence leur intérêt comme substances à activités thérapeutiques.

### Etude de la liaison membranaire vis-à-vis d'une population de récepteurs ω (benzodiazépiniques) associée aux récepteurs GABA_{A} contenant la sous-unité α₅.

Ces récepteurs peuvent être marqués sélectivement dans des membranes d'hippocampe de rat incubées en présence de [³H]flumazénil et de zolpidem 5 µM (afin de masquer les autres sous-types de récepteurs ω).

Les composés ont fait l'objet d'une étude *in vitro* quant à leur affinité pour ces récepteurs marqués par le [³H]flumazénil.

Les animaux utilisés sont des rats mâles OFA (Iffa Credo) de 200 à 250 g. Après décapitation on prélève l'hippocampe et on le broie au moyen d'un appareil Ultra-Turrax™ ou Polytron™ pendant 20 s à 6/10 de la vitesse maximale dans 80 volumes de tampon Tris 50 mM à un pH ajusté à 7,4 avec de l'acide chlorhydrique, et contenant 120 mM de chlorure de sodium et 5 mM de chlorure de potassium (5 mM).

La liaison avec le [³H]flumazénil (1 nM ; activité spécifique : 80-87 Ci/mmole ; Du Pont de Nemours / New England Nuclear) est déterminée par incubation de 200 µl de suspension de membranes dans un volume final de 1 ml de tampon contenant 5 µM de zolpidem et le composé à tester. Après une incubation de 45 min à 0°C on récupère les membranes par filtration sur filtres Whatman GF/B™ qu'on lave deux fois avec 5 ml de tampon glacé. On mesure la quantité de radioactivité retenue par le filtre par scintigraphie liquide.

La liaison spécifique du [³H]flumazénil est définie comme la quantité de radioactivité retenue sur les filtres et pouvant être inhibée par co-incubation avec le flunitrazepam 1 µM.

Pour chaque concentration de composé étudiée on détermine le pourcentage d'inhibition de la liaison du [³H]flumazénil, puis la concentration CI₅₀, concentration qui inhibe 50% de la liaison spécifique.

Les composés de l'invention les plus actifs dans cet essai ont une CI₅₀ de l'ordre de 10 à 400 nM.

### Etude des liaisons membranaires vis-à-vis des récepteurs ω₂ (benzodiazépiniques de type II) associés aux récepteurs GABA_{A} contenant majoritairement les sous-unités α₂ et α₃.

L'affinité des composés pour les récepteurs ω₂ de la moëlle épinière a été déterminée selon une variante de la méthode décrite par S. Z. Langer et S. Arbilla dans *Fund. Clin. Pharmacol.* (1988) **2** 159-170, avec utilisation de [³H]flumazénil au lieu de [³H]diazepam comme radioligand.

On homogénéise le tissu de la moëlle épinière pendant 60 s dans 30 volumes de tampon glacé (50 ml Tris/HCl, pH 7,4, NaCl 120 mM, KCl 5 mM) puis, après dilution à 1/3, on fait incuber la suspension avec du [³H]flumazénil (activité spécifique : 78 Ci/mmole ; New England Nuclear) à une concentration de 1 nM et avec les composés de l'invention, à différentes concentrations, dans un volume final de 525 µl. Après 30 min d'incubation à 0°C on filtre les échantillons sous vide sur des filtres Whatman GF/B™ et on les lave immédiatement avec du tampon glacé. La liaison spécifique du [³H]flumazénil est déterminée en présence de diazepam 1 µM non marqué. On analyse les données selon les méthodes usuelles et on calcule la CI₅₀, concentration qui inhibe 50% de la liaison du [³H]flumazénil.

Les CI₅₀ des composés de l'invention se situent, dans cet essai, entre 0,05 et 10 µM.

### Etude des liaisons membranaires vis-à-vis des récepteurs ω₁ (benzodiazépiniques de type I) associés aux récepteurs GABA_{A} contenant la sous-unité α₁.

L'affinité des composés pour les récepteurs ω₁ du cervelet a été déterminée selon une variante de la méthode décrite par S. Z. Langer et S. Arbilla dans *Fund. Clin. Pharmacol.* (1988) **2** 159-170, avec utilisation de [³H]flumazénil au lieu de [³H]diazepam comme radioligand.

On homogénéise le tissu du cervelet pendant 60 s dans 120 volumes de tampon glacé (50 ml Tris/HCl, pH 7,4, NaCl 120 mM, KCl 5 mM) puis, après dilution à 1/3, on fait incuber la suspension avec du [³H]flumazénil (activité spécifique : 78 Ci/mmole ; New England Nuclear) à une concentration de 1 nM et avec les composés de l'invention, à différentes concentrations, dans un volume final de 525 µl. Après 30 min d'incubation à 0°C on filtre les échantillons sous vide sur des filtres Whatman GF/B™ et on les lave immédiatement avec du tampon glacé. La liaison spécifique du [³H]flumazénil est déterminée en présence de diazepam 1 µM non marqué. On analyse les données selon les méthodes usuelles et on calcule la CI₅₀, concentration qui inhibe 50% de la liaison du [³H]flumazénil.

Les CI₅₀ des composés de l'invention se situent, dans cet essai, entre 0,1 et 10 µM.

Les résultats des essais effectués sur les composés de l'invention montrent que *in vitro,* ils déplacent sélectivement le [³H]flumazénil de ses sites de liaison membranaire vis-à-vis d'une population de récepteurs ω (benzodiazépiniques) associée aux récepteurs GABA_{A} contenant la sous-unité α₅, comparativement aux sous-types de récepteurs ω₁ associés aux récepteurs GABA_{A} contenant la sous-unité α₁, et comparativement à une population de récepteurs ω₂ (benzodiazépiniques de type II) associée aux récepteurs GABA_{A} contenant majoritairement les sous-unités α_{z} et α₃.

En d'autres termes, les composés ont une affinité
- forte pour les sites de liaison membranaire du [³H]flumazénil vis-à-vis d'une population de récepteurs ω (benzodiazépiniques) associée aux récepteurs GABA_{A} contenant la sous-unité α₅,
- moyenne ou faible pour les sous-types de récepteurs ω₁ (benzodiazépiniques de type I) associés aux récepteurs GABA_{A} contenant la sous-unité α₁,
- moyenne ou faible pour une population de récepteurs ω₂ (benzodiazépiniques de type II) associée aux récepteurs GABA_{A} contenant majoritairement les sous-unités α₂ et α₃.

La sélectivité représentée par le rapport CI₅₀ ω₁-cervelet / CI₅₀ ω-hippocampe est comprise entre 5 et 25 et celle représentée par le rapport CI₅₀ ω₂-moëlle / CI₅₀ ω-hippocampe est également comprise entre 5 et 25.

Les composés de l'invention peuvent être utilisés dans le traitement des affections liés aux désordres de la transmission GABAergique des récepteurs GABA_{A} associés à la sous-unité α₅. La distribution préférentielle des récepteurs ω, associés à la sous-unité α₅ du complexe récepteur GABA_{A}, dans le bulbe olfactif, dans des structures limbiques comme l'hippocampe et l'hypothalamus, et dans la moëlle épinière, suggère que les composés de l'invention peuvent être utilisés dans le traitement des troubles de l'olfaction, des troubles cognitifs, des troubles hormonaux liés au dysfonctionnement de l'hypothalamus, certains troubles émotionnels et de perception de la douleur. Ils peuvent également être utilisés dans le traitement de la spasticité et des contractures musculaires.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

1. Composé répondant à la formule générale (I) 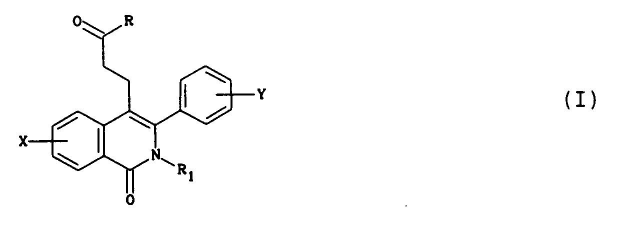 dans laquelle
X représente un atome d'hydrogène, un atome d'halogène, un groupe trifluorométhyle, un groupe alkyle en C₁-C₃, ou un ou deux groupes alcoxy en C₁-C₃,
Y représente un atome d'hydrogène, un atome d'halogène, un groupe alkyle en C₁-C₃ ou un groupe alcoxy en C₁-C₃,
R₁ représente un groupe alkyle en C₁-C₄, et
R représente un groupe hydroxy, un groupe méthoxy, un groupe éthoxy, ou un groupe de formule générale NR₂R₃ dans laquelle
R₂ et R₃ représentent chacun un atome d'hydrogène ou un groupe alkyle en C₁-C₄.

2. Composé selon la revendication 1, caractérisé en ce que R₁ représente un groupe méthyle et R représente un groupe méthylamino.

3. Procédé de préparation de composés selon la revendication 1, caractérisé en ce qu'on soumet une 3-phénylisoquinoléin-1(2*H*)-one de formule générale (II) 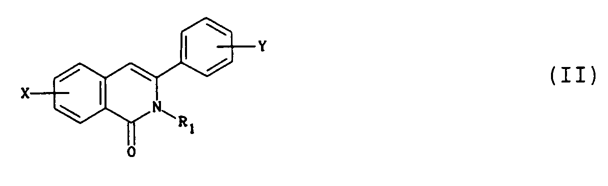 dans laquelle X, Y et R₁ sont tels que définis dans la revendication 1, à un traitement par l'oxychlorure de phosphore pour obtenir l'aldéhyde de formule générale (III) 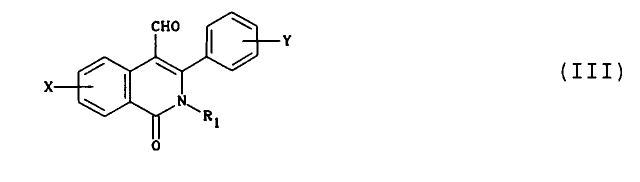 puis on traite ce dernier avec le (diméthoxyphosphinyl)acétate de méthyle pour obtenir l'ester méthylique de formule générale (IV) 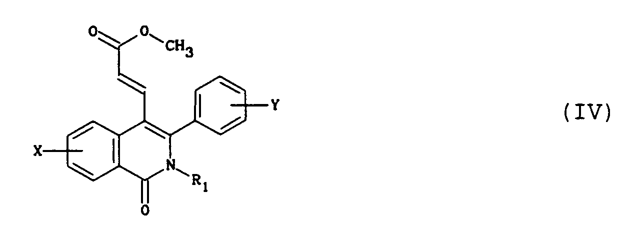 puis on soumet ce dernier à une hydrogénation pour aboutir à l'ester de formule générale (Ia) 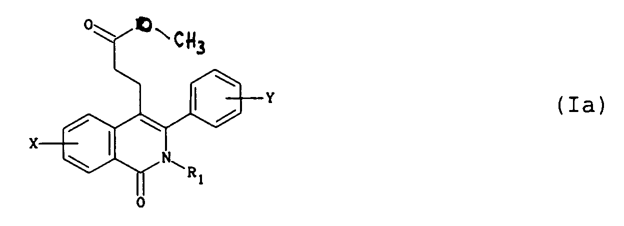 laquelle correspond à la formule générale (I) lorsque R représente un groupe méthoxy,
puis, si l'on désire un composé de formule générale (L) où R représente un groupe hydroxy, on soumet l'ester de formule générale (Ia) à une hydrolyse pour obtenir un composé de formule générale (Ib) 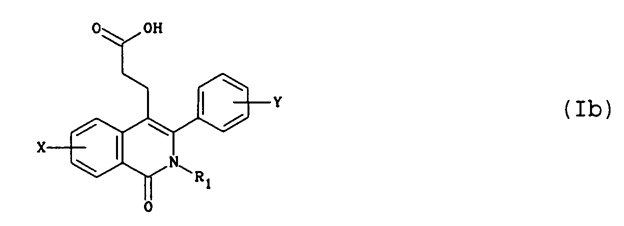 et, lorsqu'on désire un composé de formule générale (I) où R représente un groupe de formule NR₂R₃
• soit on traite l'ester de formule générale (Ia) avec une amine de formule générale HNR₂R₃,
• soit on traite l'acide de formule générale (Ib) avec cette amine en passant par l'imidazolide intermédiaire préparé *in situ* au moyen de *N,N'*-carbonyldiimidazole,
et, lorsqu'on désire un composé de formule générale (I) où R représente un groupe éthoxy, on soumet l'acide de formule générale (Ib) à l'action du chlorure de thionyle dans l'éthanol.

4. Médicament caractérisé en ce qu'il consiste en un composé selon la revendication 1 ou 2.

5. Composition pharmaceutique caractérisée en ce qu'elle contient un composé selon la revendication 1 ou 2, associé à un excipient.

## Claims

1. Compound corresponding to the general formula (I) 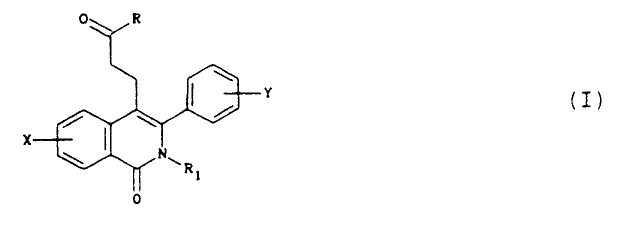 in which
X represents a hydrogen atom, a halogen atom, a trifluoromethyl group, a C₁-C₃ alkyl group or one or two C₁-C₃ alkoxy groups,
Y represents a hydrogen atom, a halogen atom, a C₁-C₃ alkyl group or a C₁-C₃ alkoxy group,
R₁ represents a C₁-C₄ alkyl group, and
R represents a hydroxyl group, a methoxy group, an ethoxy group or a group of general formula NR₂R₃ in which R₂ and
R₃ each represent a hydrogen atom or a C₁-C₄ alkyl group.

2. Compound according to Claim 1, characterized in that R₁ represents a methyl group and R represents a methylamino group.

3. Process for the preparation of compounds according to Claim 1, characterized in that a 3-phenylisoquinol-1(2*H*)-one of general formula (II) 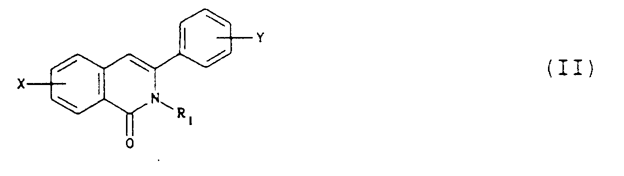 in which X, Y, and R₁ are as defined in Claim 1, is treated with phosphorus oxychloride in order to obtain the aldehyde of general formula (III) 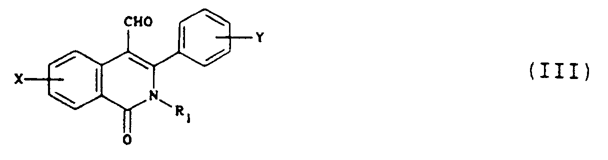 the latter is then treated with methyl (dimethoxyphosphinyl)acetate in order to obtain the methyl ester of general formula (IV) 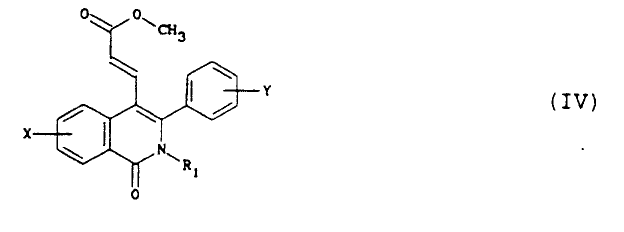 the latter is then hydrogenated in order to result in the ester of general formula (Ia) 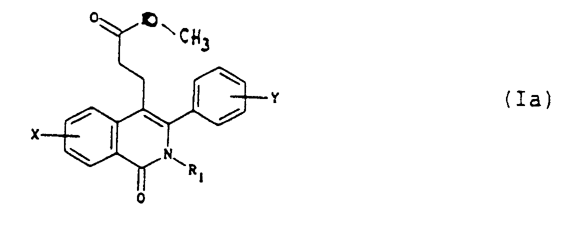 which corresponds to the general formula (I) when R represents a methoxy group,
then, if a compound of general formula (I) where R represents a hydroxyl group is desired, the ester of general formula (Ia) is hydrolysed in order to obtain a compound of general formula (Ib) 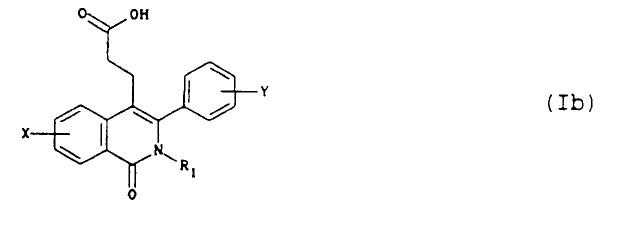 and, when a compound of general formula (I) where R represents a group of formula NR₂R₃ is desired,
• either the ester of general formula (Ia) is treated with an amine of general formula HNR₂R₃,
• or the acid of general formula (Ib) is treated with this amine via the intermediacy of the imidazolide prepared *in situ* using *N*,*N*'-carbonyldiimidazole,
and, when a compound of general formula (I) where R represents an ethoxy group is desired, the acid of general formula (Ib) is reacted with thionyl chloride in ethanol.

4. Medicament, characterized in that it comprises a compound according to Claim 1 or 2.

5. Pharmaceutical composition, characterized in that it contains a compound according to Claim 1 or 2, in combination with an excipient.

## Patentansprüche

1. Verbindung der allgemeinen Formel (I) 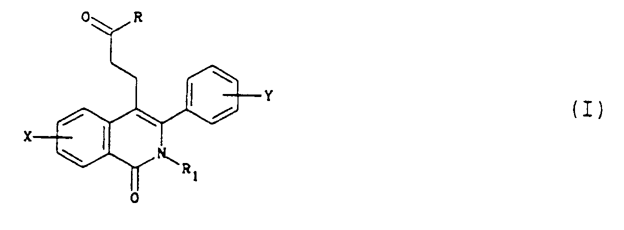 in der
X ein Wasserstoffatom, ein Halogenatom, eine Trifluormethylgruppe, eine C₁-C₃-Alkylgruppe oder eine oder zwei C₁-C₃-Alkoxygruppen,
Y ein Wasserstoffatom, ein Halogenatom, eine C₁-C₃-Alkylgruppe oder eine C₁-C₃-Alkoxygruppe.
R₁ eine C₁-C₄-Alkylgruppe und
R eine Hydroxygruppe, eine Methoxygruppe, eine Ethoxygruppe oder eine Gruppe der allgemeinen Formel NR₂R₃, in der R₂ und R₃ jeweils ein Wasserstoffatom oder eine C₁-C₄-Alkylgruppe darstellen,
bedeuten.

2. Verbindung nach Anspruch 1, dadurch gekennzeichnet, daß R₁ eine Methylgruppe und R eine Methylaminogruppe bedeuten.

3. Verfahren zur Herstellung der Verbindungen nach Anspruch 1, dadurch gekennzeichnet, daß man ein 3-Phenylisochinolin-1(2H)-on der allgemeinen Formel (II) 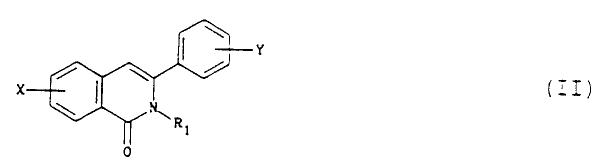 in der X, Y und R₁ die in Anspruch 1 angegebenen Bedeutungen besitzen, einer Behandlung mit Phosphoroxidchlorid unterwirft zur Bildung des Aldehyds der allgemeinen Formel (III) 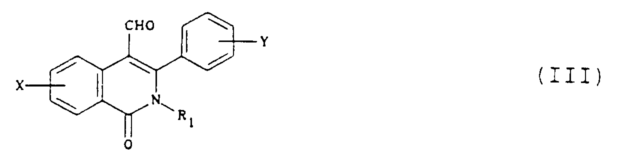 und dann diesen letzteren mit (Dimethoxyphosphinyl)-essigsäuremethylester behandelt zur Bildung des Methylesters der allgemeinen Formel (IV) 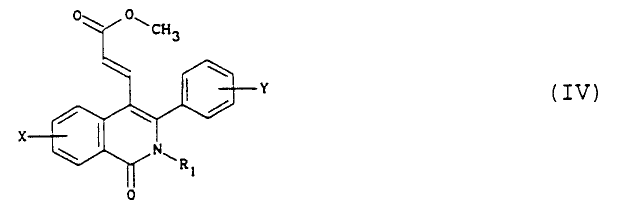 welchen man einer Hydrierung unterzieht zur Bildung des Esters der allgemeinen Formel (Ia) 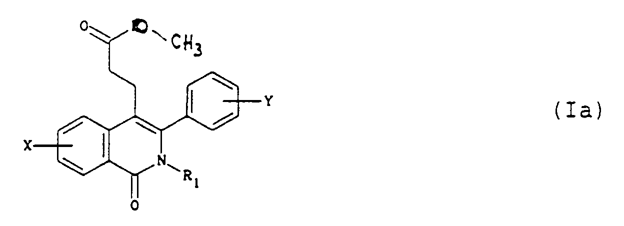 welcher der allgemeinen Formel (I) entspricht, wenn R eine Methoxygruppe darstellt,
wonach man, wenn man eine Verbindung der allgemeinen Formel (I) herzustellen wünscht, In der R eine Hydroxygruppe darstellt, den Ester der allgemeinen Formel (Ia) einer Hydrolyse unterzieht zur Bildung einer Verbindung der allgemeinen Formel (Ib) 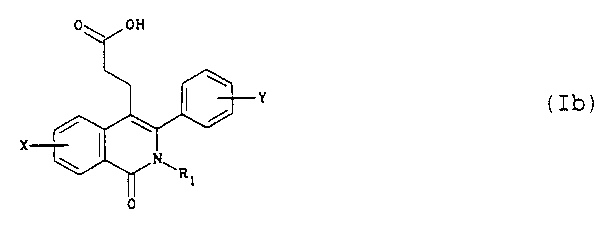 und, wenn man eine Verbindung der allgemeinen Formel (I) herzustellen wünscht, in der R eine Gruppe der Formel NR₂R₃ darstellt, man
• entweder den Ester der allgemeinen Formel (Ia) mit einem Amin der allgemeinen Formel HNR₂R₃ behandelt,
• oder die Saure der allgemeinen Formel (Ib) mit diesem Amin behandelt, wobei man die Reaktionüber das Imidazolid-Zwischenprodukt fuhrt, welches in situ mit N,N'-Carbonyldiimidazol gebildet wird,
und, wenn man eine Verbindung der allgemeinen Formel (I) herzustellen wünscht, in der R eine Ethoxygruppe darstellt, man die Säure der allgemeinen Formel (Ib) der Einwirkung von Thionylchlorid in Ethanol unterwirft.

4. Arzneimittel, dadurch gekennzeichnet, daß es aus einer Verbindung nach Anspruch 1 oder 2 besteht.

5. Pharmazeutische Zubereitung, dadurch gekennzeichnet, daß sie eine Verbindung nach Anspruch 1 oder 2 in Kombination mit einem Trägermaterial enthält.
